(19) Europäisches Patentamt
European Patent Office
Office européen des brevets

(11) EP 0 513 627 B1

(12) **EUROPÄISCHE PATENTSCHRIFT**

(45) Veröffentlichungstag und Bekanntmachung des Hinweises auf die Patenterteilung:
**28.02.1996 Patentblatt 1996/09**

(51) Int Cl.$^6$: **C11B 9/00**, A23L 1/226, C07D 309/30

(21) Anmeldenummer: **92107551.1**

(22) Anmeldetag: **05.05.1992**

(54) **Tetrahydro-alpha-pyronderivat, Verfahren zu seiner Herstellung und dieses enthaltende Riech- und/oder Geschmackstoffkompositionen**

Tetrahydro-alpha-pyrone derivative, method for its preparation and perfume and/or flavouring compositions containing it

Dérivé de tétrahydro-alpha-pyrone, procédé pour sa préparation et compositions parfumantes et/ou aromatisantes le contenant

(84) Benannte Vertragsstaaten:
**CH DE ES FR GB IT LI NL**

(30) Priorität: **15.05.1991 CH 1450/91**
**18.03.1992 CH 876/92**

(43) Veröffentlichungstag der Anmeldung:
**19.11.1992 Patentblatt 1992/47**

(73) Patentinhaber:
**GIVAUDAN-ROURE (INTERNATIONAL) S.A.**
**CH-1214 Vernier, Genève (CH)**

(72) Erfinder: **Kaiser, Roman**
**CH-8610 Uster (CH)**

(74) Vertreter: **Urech, Peter, Dr. et al**
**CH-4002 Basel (CH)**

(56) Entgegenhaltungen:
- **PATENT ABSTRACTS OF JAPAN vol. 9, no. 12 (C-281)(1835) 16. Mai 1985**
- **TETRAHEDRON LETTERS, Bd. 24, Nr. 18, Mai 1983, Oxford, GB, Seiten 1893 - 1896;J.R. ROCCA ET AL.: 'Synthesis and stereochemistry of tetrahydro-3,5-dimethyl-6-(1 methylbutyl)2H-pyran-2-one, a component of the queen recognition pheromoneof Solenopsis invicta'**
- **JOURNAL OF ORGANIC CHEMISTRY, Bd. 54, Nr. 16, 4. August 1989, Washington, DC, US, Seiten 3988 - 3992; F. KAZMIERCZAK ET AL.: 'Synthesis of the branched nine- carbon unit of the type A streptogramins and other antibiotics'**
- **JOURNAL OF THE CHEMICAL SOCIETY, PERKIN TRANSACTIONS 1, Nr. 4, April 1991, Letchworth, GB, Seiten 667 - 692; S.V. LEY ET AL.: 'Total synthesis of the anthelmintic macrolide avermectin B1a'**
- **TETRAHEDRON LETTERS, Bd. 32, Nr. 14, 1. April 1991, Oxford, GB, Seiten 1719 - 1722; J. SHERKENBECK ET AL.: 'Synthesis and structure-activity relationships of some mibemycin E analogues'**
- **JOURNAL OF THE AMERICAN CHEMICAL SOCIETY, Bd. 105, Nr. 23, 16. November 1983, Washington, DC, US, Seiten 6882 - 6889; D.B. COLLUM ET AL.: 'Mercury(II)- mediated opening of cyclopropanes. Effects of proximate internal nucleophileson stereo- and regioselectivity'**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein neues Tetrahydro-α-pyronderivat, nämlich das neue 5-Methyl-6-pentyl-tetrahydro-α-pyron (4-Methyl-5-decanolid) der Formel

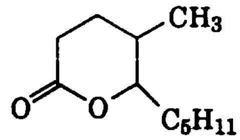

I

dessen Herstellung, Riechstoff- und Geschmackstoffkompositionen, die diese Verbindung I als organoleptischen Wirkstoff enthalten, sowie die Verwendung der Verbindung I als Riech- und/oder Geschmackstoff.

Aus Tetrahedron Letters 24 (1983) 1893-1896 ist die Verbindung der Formel

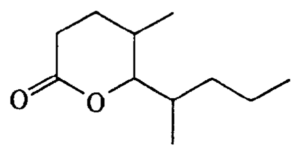

bekanntgeworden, aus Journal of Organic Chemistry, 54 (1989) 3988-3992 ist die Verbindung der Formel

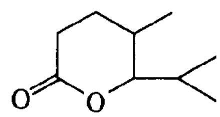

bekanntgeworden.

Die Publikationen enthalten keinen diesbezüglichen Hinweis auf die organoleptischen Eigenschaften dieser Substanzen, ein solcher liesse sich dem experimentellen Teil dieser Arbeiten auch nicht entnehmen.

Patent Abstracts ofJapan, Vol. 9, No. 12 (C-281) (1985), 16. Mai 1985 beschreibt die Verwendung der Verbindung der Formel

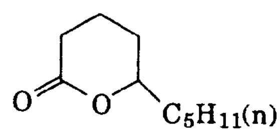

als Aromastoff für Nahrungsmittel.

Die Verbindung der Formel I kann u.a. angesichts des Methylrestes in der 5-Stellung des Tetrahydro-α-pyronringes als Stereoisomere vorliegen. Die Formel I soll u.a. diese stereoisomeren Formen und deren Gemische umfassen.

Es hat sich herausgestellt, dass das eine untenstehende Stereoisomere (das cis- oder trans-Isomere) eines stereoisomeren Paares der Formel I, verglichen mit dem andern Stereoisomeren, unterschiedliche organoleptische Eigenschaften besitzt:

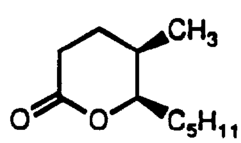

I'a

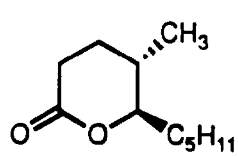

I'b

Das cis-Isomere I'a besitzt einen sehr angenehmen und überraschend raumfüllenden Geruch, der einerseits an bestimmte Aspekte der Blütendüfte der Tuberose (Polianthes tuberosa) und Gardenia-Arten, andererseits an Caramel, Kondensmilch und Kokos, insbesondere Kokosmilch, erinnert. Das trans-Isomere zeigt ähnliche olfaktorische Charakteristiken.

Das erfindungsgemässe Verfahren zur Herstellung des erfindungsgemässen 5-Methyl-6-pentyl-tetrahydro-α-pyrons (4-Methyl-5-decanolids) ist dadurch gekennzeichnet, dass man 3-Methyl-2-pentyl-cyclopentan-1-on einer Bayer-Villiger-Oxidation unterwirft (gemäss der Gleichung

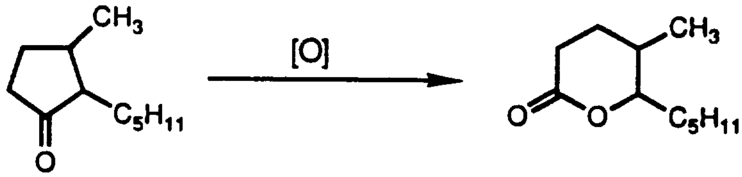

)

II I'

Als Oxidationsmittel verwendet man eine Persäure, beispielsweise Peressigsäure, Trifluorperessigsäure, Perbenzoesäure oder Monoperphthalsäure, von denen Peressigsäure das bevorzugte Oxidationsmittel darstellt. Die Umsetzung erfolgt zweckmässigerweise in einem inerten organischen Verdünnungsmittel, wie Methylenchlorid, Dichloräthan, Toluol, Xylol, etc. bei Temperaturen zwischen -30°C und +50°C, vorzugsweise im Temperaturbereich von 20°C bis 35°C.

Als Ausgangsmaterial kann man reines cis- oder trans-3-Methyl-2-pentyl-cyclopentan-1-on verwenden, was vorwiegend zum cis- bzw. trans-5-Methyl-6-pentyl-tetrahydro-α-pyron (4-Methyl-5-decanolid) führt. Zweckmässigerweise geht man jedoch von einem cis-/trans-Gemisch des 2-Pentyl-3-methyl-cyclopentan-1-on aus, da ein solches cis-/trans-Gemisch geeigneterweise durch auf an sich bekannte Weise durchführbare Hydrierung des als Parfümerieprodukt bekannten 3-Methyl-2-pentyl-2-cydopenten-1-ons (Dihydrojasmon) der Formel

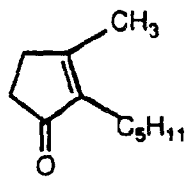

erhältlich ist. Je nach Reaktionsbedingungen (Lösungsmittel, Temperatur, Katalysator usw.) bildet sich aus dem durch die Hydrierung primär erhaltenen cis-2-Pentyl-3-methyl-cyclopentan-1-on mehr oder weniger die entsprechende trans-Verbindung.

Im Falle des Anfallens eines cis-/trans-Gemisches des 5-Methyl-6-pentyl-tetrahydro-α-pyrons (Verbindung der Formel I) kann dieses nach an sich bekannten Methoden in die reinen cis- und trans-Isomeren aufgetrennt werden, beispielsweise durch chromatographische oder destillative Verfahren.

Die Ausbeuten an olfaktorisch gutem Material sind für beide Reaktionsschritte Hydrierung und Oxidation hoch, normalerweise über 75%.

Die erfindungsgemässe Verbindung I [das cis-Isomere I'a, das trans-Isomere I'b sowie deren Gemische (cis-/trans-)] zeichnet sich im allgemeinen durch Duftnoten aus, die einerseits an bestimmte Aspekte der Blütendüfte der Tuberose und Gardenia-Arten, Kondensmilch, Caramel und Kokos, andererseits an solche tropischer Früchte erinnern.

Aufgrund ihrer obenerwähnten wertvollen olfaktorischen Eigenschaften eignet sich die Verbindung I als Riech- und/oder Geschmackstoff, und zwar insbesondere in Kombination mit der heutzutage zur Verfügung stehenden umfangreichen Palette von natürlichen und synthetischen Riechstoffe bzw. Geschmackstoffe zur Kreation von Parfüm- bzw. Aroma-Kompositionen, welche ihre Anwendung in allen üblichen Applikations-Segmenten finden können. Beispiele der zahlreichen bekannten Riechstoffingredientien natürlichen oder synthetischen Ursprungs, wobei die Palette der natürlichen Rohstoffe sowohl leicht- als auch mittel- und schwerflüchtige Komponenten, und diejenige der Synthetika Vertreter aus etlichen Stoffklassen umfassen kann, sind:

- Naturprodukte, wie Baummoos-Absolue, Basilikumöl, Agrumenöle (wie Bergamotteöl, Mandarinenöl, usw.), Mastix-Absolue, Myrtenöl, Palmarosaöl, Patchouliöl, Petitgrainöl, Wermutöl, Lavendelöl, Rosenöl, Jasminöl, Ylang-Ylangöl, Sandelholzöl,

- Alkohole, wie Farnesol, Geraniol, Linalool, Nerol, Phenyläthylalkohol, Rhodinol, Zimtalkohol, cis-3-Hexenol, Menthol, α-Terpineol,

- Aldehyde, wie Citral, α-Hexylzimtaldehyd, Hydroxycitronellal, Lilial® (p-tert.Butyl-α-methyl-dihydrozimtaldehyd), Methylnonylacetaldehyd, Phenylacetaldehyd, Anisaldehyd, Vanillin,

- Ketone, wie Allyljonon, α-Jonon, β-Jonon, Isoraldein (Isomethyl-α-jonon), Verbenone, Nootkaton, Geranylaceton,

- Ester, wie Allyl-phenoxyacetat, Benzyl-salicylat, Cinnamylpropionat, Citronellyl-acetat, Decylacetat, Dimethylbenzylcarbinyl-acetat, Aethylacetoacetat, Aethyl-acetylacetat, cis-3-Hexenyl-isobutyrat, Linalylacetat, Methyl-dihydrojasmonat, Styrallylacetat, Vetiverylacetat, Benzylacetat, cis-3-Hexenylsalicylat, Geranylacetat, usw.

- Lactone, wie γ-Undecalacton, δ-Decalacton, Pentadecan-15-olid,

- verschiedene in der Parfümerie oft benützte Komponenten, wie Indol, p-Menthan-8-thiol-3-on, Methyleugenol, Eugenol, Anethol.

Die unter Verwendung der Verbindung I hergestellten Riechstoffkompositionen, insbesonders solcher blumiger, blumig-würziger, blumig-fruchtiger und blumig-orientalischer Richtung, bestechen besonders durch ihre Originalität.

Bei ihrer Verwendung als Riechstoffe fässt sich die Verbindung der Formel I (bzw. deren Gemische) in weiten Grenzen einsetzen, die beispielsweise von ca. 0,1 (Detergentien) bis ca. 30 Gewichtsprozent (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen ca. 0,5 und ca. 10 Gewichtsprozent. Die mit der Verbindung I hergestellten Kompositionen lässt sich für alle Arten von parfümieren Verbrauchsgütern einsetzen (Eaux de Cologne, Eaux de Toilette, Extraits, Lotionen, Cremes, Shampoos, Seifen, Salben, Puder, Zahnpasten, Mundwässer, Desodorantien, Detergentien, Gewebeveredler, Tabak, usw.).

Die Verbindung I kann demgemäss bei der Herstellung von Kompositionen und - wie obige Zusammenstellung zeigt - unter Verwendung einer breiten Palette bekannter Riechstoffe bzw. Riechstoffgemische verwendet werden. Bei der Herstellung solcher Kompositionen können die oben aufgeführten bekannten Riechstoffe bzw. Riechstoffgemische nach (dem Parfümeur) bekannter Art und Weise verwendet werden, wie dies z.B. aus W.A. Poucher, Perfumes, Cosmetics and Soaps 2, 7. Auflage, Chapman und Hall, London, 1974 hervorgeht.

Als Geschmackstoffe kann die Verbindung I beispielsweise zur Erzeugung bzw. Verbesserung, Verstärkung, Steigerung oder Modifizierung von Fruchtaromen, z.B. Mango, Passionsfrucht, Pfirsich, Kokos verwendet werden. Als Anwendungsgebiete dieser Aromen kommen beispielsweise Nahrungsmittel (Joghurt, Süsswaren, Desserts, insbesondere Desserts auf Caramelbasis, usw.), Genussmittel (Tee, Kaffee, Tabak, usw.) und Getränke (Limonade usw.) in Frage.

Die ausgeprägten geschmacklichen Qualitäten der Verbindung I ermöglichen die Verwendung als Geschmackstoff in geringen Konzentrationen. Eine geeignete Dosierung umfasst den Bereich von 0,01 bis 100 ppm, vorzugsweise von 0,1 bis 10 ppm im Fertigprodukt, d.h. dem aromatisierten Nahrungsmittel, Genussmittel oder Getränk.

Die Verbindung kann auf übliche Weise mit den für Geschmackstoffkompositionen verwendeten Bestandteilen vermischt bzw. solchen Aromen zugesetzt werden. Unter den erfindungsgemäss verwendeten Aromen werden Geschmackstoffkompositionen verstanden, die sich auf an sich bekannte Art verdünnen bzw. in essbaren Materialien verteilen lassen. Sie enthalten beispielsweise etwa 0,01 -30, insbesondere 0,1 - 10 Gew.% Geschmackstoff(e) der Formel I. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Die bei der Herstellung solcher Aromen zweckmässigerweise verwendeten bekannten Aromastoffe sind entweder in der obigen Zusammenstellung bereits enthalten oder können leicht der Literatur entnommen werden, wie z.B. J. Merory, Food Flavorings, Composition, Manufacture and Use, Second Edition, The Avi Publishing Company, Inc., Westport, Conn. 1968, oder G. Fenaroli, Fenaroli's Handbook of Flavor Ingredients, Second Edition, Volume 2, CRC-Press, Inc. Cleveland, Ohio, 1975.

Für die Herstellung solcher üblicher Gebrauchsformen kommen beispielsweise folgende Trägermaterialien, Verdikkungsmittel, Geschmackstoffverbesserer, Gewürze und Hilfsingredientien, usw. in Frage:

Gummi arabicum, Tragant, Salze oder Brauereihefe, Alginate, Carrageen oder ähnliche Absorbentien; Maltol, Gewürzoleoresine, Raucharomen; Gewürznelken, Diacetyl, Natriumcitrat; Mononatriumglutamat, Dinatriuminosin-5'-monophosphat (IMP), Dinatriumguanosin-5-phosphat (GMP); oder spezielle Aromastoffe, Wasser, Aethanol, Propylenglykol, Glycerin, usw.

Dank ihrer hochstehenden olfaktorischen Eigenschaften wird die Verbindung der Formel I bevorzugt in der Luxusparfümerie und in Kompositionen für Kosmetika zum Einsatz kommen.

Die nachstehenden Beispiele illustrieren die Erfindung.

I. Herstellung der Verbindungen der Formel I, I'a und I'b

Beispiel 1

78,0 g (0,47 Mol) kommerziell erhältliches 3-Methyl-2-pentyl-2-cyclopenten-1-on (Dihydrojasmon) werden in 90 ml reinem Aethanol gelöst, mit 0,50 g Palladium 10% auf Aktivkohle versetzt und anschliessend unter intensivem Rühren bei Normaldruck und Raumtemperatur bis zur Sättigung hydriert (verbrauchter Wasserstoff: 12,0 l in 2 Stunden). Die Reaktionslösung wird durch Filtration vom Katalysator befreit und anschliessend unter vermindertem Druck eingeengt. Die Destillation des Rohproduktes (76,0 g) über eine Destillationskolonne ergibt 68,0 g Material vom $Kp_{0,12}$ 67-68°C, welches bei einer Ausbeute von über 98% cis-3-Methyl-2-pentyl-cyclopentan-1-on (IIa) und trans-3-Methyl-2-pentyl-cyclopentan-1-on (IIb) im Verhältnis von 3:2 enthält.

42,6 g (0,25 Mol) des obigen Gemisches von IIa und IIb werden in 200 ml Methylenchlorid gelöst, mit 34,0 g wasserfreiem Natriumacetat versetzt und anschliessend unter gutem Rühren und Eiskühlung im Verlaufe von 30 Minuten mit 62,5 g Peressigsäure (40%) versetzt. Anschliessend wird die Eiskühlung entfernt und die Reaktionslösung durch gelegentliches Kühlen mit Wasser bei 15°C in einem Temperaturbereich von 25-30°C gehalten.

Nach 1,5 Stunden kann keine exotherme Reaktion mehr festgestellt werden, und eine gaschromatographische Analyse zeigt den gewünschten Umsatz von über 95% an. Die Reaktionslösung wird nun dreimal mit Wasser, dreimal mit Natriumsulfit-Lösung (10%) und zweimal mit Kochsalzlösung gewaschen, und die organische Phase mit wasserfreiem Natriumsulfat getrocknet und anschliessend unter vermindertem Druck eingeengt.

Die Destillation des so erhaltenen Rohlactones (41,9 g) über eine Destillationskolonne ergibt 35,9 g (78%) olfaktorisch gutes Produkt vom $Kp_{0,05}$ 82-83°C, bestehend aus über 97% cis-4-Methyl-5-decanolid (I'a) und trans-4-Methyl-5-decanolid (I'b) im Verhältnis von ca. 3:2.

Die im folgenden durch ihre spektralen Daten charakterisierten Isomeren I'a und I'b werden durch Auftrennung des oben beschriebenen Gemisches mit Hilfe der Gaschromatographie erhalten. Das Isomere I'a zeigt auf den üblichen gaschromatographischen Säulen eine etwas längere Retentionszeit als das Isomere I'b.

cis-4-Methyl-5-decanolid (I'a):
Infrarot-Spektrum: 1735, 1238, 1200, 1140, 1095, 1069, 1054, 993, 908 $cm^{-1}$;
$^1H$-NMR (400 MHz, $CDCl_3$): 0,90 (t,J~7, $-CH_3$), 0,96 [d,J~7, $H_3C$-C(4)], 1,25-1,60 (m, zusammen 6H), 1,62-1,72 (m,2H), 1,95-2,10 (m,3H), 2,53 [dxd, J~7, 2H-C(2)],4,28 [m, H-C(5)] ppm;
Massenspektrum (m/e): ($M^+$, 0,2), 128(3), 113(23), 99(3), 95(1), 85(15), 84(39), 69(5), 67,5(5), 57(10), 56(100), 55 (24), 43(26), 41(22).
trans-4-Methyl-5-decanolid (I'b):
Infrarot-Spektrum: 1735, 1248, 1200, 1118, 1098, 1080, 1032, 2020 $cm^{-1}$; $^1H$-NMR (400 MHz, $CDCl_3$): 0,90 (t,J~7, $-CH_3$), 1,00 [d,J~7, $H_3C$-C(4)], 1,22-1,64 (m, zusammen 8H), 1,66-1,78 (m,2H), 1,86-1,94 (m,1H), 2,42-2,51 [m, $H_a$-C(2)], 2,58-2,65 [m, $H_b$-C(2)], 3,93 [m, H-C(5)] ppm;
Massenspektrum (m/e): 184 ($M^+$, 0,2), 128(4), 114(10), 113(100), 99(3), 95(2), 85(27), 84(44), 69(8), 67(8), 57(14), 56(100), 55(34), 43(29), 41(26).

II. Formulierungsbeispiele

Beispiel 2

Parfümerie-Akkord (Komposition) in Richtung Ylang-Ylang mit einem Gehalt an cis- und trans-4-Methyl-5-decanolid:

| | Gewichtsteile |
|---|---|
| Linalool | 150 |
| Benzylacetat | 130 |
| Benzylsalicylat | 100 |
| Cinnamylacetat | 80 |
| Geranylacetat | 80 |
| Benzylbenzoat | 80 |
| Methylbenzoat | 78 |
| Geraniol | 75 |
| Zimtalkohol | 60 |

Fortsetzung der Tabelle auf der nächsten Seite

(fortgesetzt)

| | Gewichtsteile |
|---|---|
| Thibetolid | 30 |
| cis-3-Hexenylsalicylat | 30 |
| Hydroxycitronellal | 20 |
| Eugenol | 20 |
| cis-3-Hexenylbenzoat | 20 |
| Methyljasmonat | 10 |
| β-Jonon | 5 |
| cis-3-Hexenol | 1 |
| cis-3-Hexenylacetat | 1 |
| | 970 |

Der Zusatz von 30 Gewichtsteilen des cis-/trans-Gemisches I'a/I'b (3:2) bewirkt eine sehr angenehme Abrundung dieses Parfümerie-Akkordes. Die in Richtung "Ylang-Ylang" gehende Grundnote wird durch den Frangipaniblüten (Plumeria acutifolia) und Tuberose erinnernde Aspekte bereichert. Dieses positiven Effekte lassen sich auch nach 24 Stunden noch deutlich feststellen.

Beispiel 3

Kräuterte-Akkord, verwendbare für Aromen wie auch für Parfüm-Kreationen (Kompositionen):

| | Gewichtsteile |
|---|---|
| Linalool | 210 |
| Anethol | 120 |
| Phenyläthylalkohol | 120 |
| α-Terpineol | 60 |
| Citronellol | 60 |
| Geranylacetone | 60 |
| cis-3-Hexenylbenzoat | 60 |
| Anisaldehyd | 45 |
| Phenylacetaldehyd | 30 |
| Eugenol | 30 |
| Menthol | 30 |
| Estragol | 15 |
| Verbenon | 15 |
| β-Jonon | 9 |
| Citral | 6 |
| Methyljasmonat | 3 |
| cis-3-Hexenol | 1 |
| Dipropylenglykol | 96 |
| | 970 |

Der Zusatz von 30 Gewichtsteilen des cis-/trans-Gemisches von I'a/I'b (3:2) verleiht diesem Akkord viel Wärme und Natürlichkeit. Eine Note, die mit frisch getrockneten Alpenkräutern assoziiert werden kann, kommt sehr vorteilhaft zur Geltung.

Beispiel 4

Aroma mit "Milch-Charakter":

| | a | b |
|---|---|---|
| | Gewichtsteile | |
| Acetoin | 25 | 25 |
| Vanillin | 20 | 20 |
| Maltol | 10 | 10 |
| Diacetyl | 10 | 10 |
| Aethyllactat | 10 | 10 |
| δ-Decalacton | 10 | 10 |
| Capronsäure | 7 | 7 |
| Aethylbutyrat | 4 | 4 |
| Caprylsäure | 1 | 1 |
| cis- und trans-4-Methyl-5-decanolide (I'a/I'b) | - | 10 |
| Propylenglykol | 903 | 893 |
| | 1000 | 1000 |

Die beiden Aromen a und b werden nach Verdünnen in Zuckersirup-Lösung (20 g Aroma a bzw. b pro 100 l Zuckersirup 10°Bx) vergleichend verkostet.

Aroma b wird von einem Panel von Testpersonen eindeutig vorgezogen, da die gewünschten an Sahne und Kondensmilch erinnernden Noten verstärkt zur Geltung kommen.

## Patentansprüche

**Patentansprüche für folgende Vertragsstaaten : CH, DE, FR, GB, IT, LI, NL**

**1.** 5-Methyl-6-pentyl-tetrahydro-α-pyron.

**2.** Synthetisch hergestelltes 5-Methyl-6-pentyl-tetrahydro-α-pyron.

**3.** 5-Methyl-6-pentyl-tetrahydro-α-pyron in Form seines cis-Isomeren.

**4.** Synthetisch hergestelltes cis-5-Methyl-6-pentyl-tetrahydro-α-pyron.

**5.** 5-Methyl-6-pentyl-tetrahydro-α-pyron in Form seines trans-Isomeren.

**6.** Synthetisch hergestelltes trans-5-Methyl-6-pentyl-tetrahydro-α-pyron.

**7.** Verfahren zur Herstellung von 5-Methyl-6-pentyl-tetrahydro-α-pyron, dadurch gekennzeichnet, dass man 3-Methyl-2-pentyl-cyclopentan-1-on einer Bayer-Villiger-Oxidation unterwirft.

**8.** Riechstoff- und/oder Geschmackstoffkomposition gekennzeichnet durch einen Gehalt an cis- oder trans-5-Methyl-6-pentyl-tetrahydro-α-pyron oder an einem Gemisch der beiden Isomeren.

**9.** Riechstoff- und/oder Geschmackstoffkomposition gemäss Anspruch 8, gekennzeichnet durch einen Gehalt an cis- oder trans-5-Methyl-6-pentyl-tetrahydro-α-pyron oder an einem Gemisch der beiden Isomeren, mit der Ausnahme dieses natürlich vorkommende Tetrahydro-α-pyron-derivat enthaltenden Riechstoff- oder Geschmackstoffkompositionen.

**10.** Verwendung von cis-5-Methyl-6-pentyl-tetrahydro-α-pyron, von trans-5-Methyl-6-pentyl-tetrahydro-α-pyron oder eines cis-/trans-Isomerengemisches von 5-Methyl-6-pentyl-tetrahydro-α-pyron als Riech- und/oder Geschmackstoff.

**Patentansprüche für folgenden Vertragsstaat : ES**

1. Riechstoff- und/oder Geschmackstoffkomposition gekennzeichnet durch einen Gehalt an cis- oder trans-5-Methyl-6-pentyl-tetrahydro-α-pyron oder an einem Gemisch der beiden Isomeren.

2. Riechstoff- und/oder Geschmackstoffkomposition gemäss Anspruch 8, gekennzeichnet durch einen Gehalt an cis- oder trans-5-Methyl-6-pentyl-tetrahydro-α-pyron oder an einem Gemisch der beiden Isomeren, mit der Ausnahme dieses natürlich vorkommende Tetrahydro-α-pyron-derivat enthaltenden Riechstoff- oder Geschmackstoffkompositionen.

3. Verfahren zur Herstellung von 5-Methyl-6-pentyl-tetrahydro-α-pyron, dadurch gekennzeichnet, dass man 3-Methyl-2-pentyl-cyclopentan-1-on einer Bayer-Villiger-Oxidation unterwirft.

4. Verwendung von cis-5-Methyl-6-pentyl-tetrahydro-α-pyron, von trans-5-Methyl-6-pentyl-tetrahydro-a-pyron oder eines cis-/trans-Isomerengemisches von 5-Methyl-6-pentyl-tetrahydro-α-pyron als Riech- und/oder Geschmackstoff.

**Claims**

**Claims for the following Contracting States : CH, DE, FR, GB, IT, LI, NL**

1. 5-Methyl-6-pentyl-tetrahydro-α-pyrone.

2. Synthetically manufactured 5-methyl-6-pentyl-tetrahydro-α-pyrone.

3. 5-Methyl-6-pentyl-tetrahydro-α-pyrone in the form of its cis isomer.

4. Synthetically manufactured cis-5-methyl-6-pentyl-tetrahydro-α-pyrone.

5. 5-Methyl-6-pentyl-tetrahydro-α-pyrone in the form of its trans isomer.

6. Synthetically manufactured trans-5-methyl-6-pentyl-tetrahydro-α-pyrone.

7. A process for the manufacture of 5-methyl-6-pentyl-tetrahydro-α-pyrone, characterized by subjecting 3-methyl-2-pentyl-cyclopentan-1-one to a Bayer-Villiger oxidation.

8. An odorant and/or flavorant composition, characterized in that it contains cis- or trans-5-methyl-6-pentyl-tetrahydro-α-pyrone or a mixture of the two isomers.

9. An odorant and/or flavorant composition, according to claim 8, characterized in that it contains cis- or trans-5-methyl-6-pentyl-tetrahydro-α-pyrone or a mixture of the two isomers, with the exception of odorant or flavorant compositions containing this naturally occurring tetrahydro-α-pyrone derivative.

10. The use of cis-5-methyl-6-pentyl-tetrahydro-α-pyrone, of trans-5-methyl-6-pentyl-tetrahydro-α-pyrone or of a cis/trans isomer mixture of 5-methyl-6-pentyl-tetrahydro-α-pyrone as an odorant and/or flavorant.

**Claims for the following Contracting States : ES**

1. An odorant and/or flavorant composition, characterized in that it contains cis- or trans-5-methyl-6-pentyl-tetrahydro-α-pyrone or a mixture of the two isomers.

2. An odorant and/or flavorant composition according to claim 1, characterized in that it contains cis- or trans-5-methyl-6-pentyl-tetrahydro-α-pyrone or a mixture of the two isomers, with the exception of odorant or flavorant compositions containing this naturally occurring tetrahydro-α-pyrone derivative.

3. A process for the manufacture of 5-methyl-6-pentyl-tetrahydro-α-pyrone, characterized by subjecting 3-methyl-2-pentyl-cyclopentan-1-one to a Bayer-Villiger oxidation.

4. The use of cis- 5-methyl-6-pentyl-tetrahydro-α-pyrone of trans-5-methyl-6-pentyl-tetrahydro-α-pyrone or of a cis/trans isomer mixture of 5-methyl-6-pentyl-tetrahydro-α-pyrone as an odorant and/or flavorant.

**Revendications**

**Revendications pour les Etats contractants suivants : CH, DE, FR, GB, IT, LI, NL**

1. 5-méthyl-6-pentyl-tétrahydro-α-pyrone.

2. 5-méthyl-6-pentyl-tétrahydro-α-pyrone préparée par voie de synthèse.

3. 5-méthyl-6-pentyl-tétrahydro-α-pyrone, sous forme de son isomère cis.

4. Cis-5-méthyl-6-pentyl-tétrahydro-α-pyrone préparée par voie de synthèse.

5. 5-méthyl-6-pentyl-tétrahydro-α-pyrone, sous forme de son isomère trans.

6. Trans-5-méthyl-6-pentyl-tétrahydro-α-pyrone, préparée par voie de synthèse.

7. Procédé pour préparer la 5-méthyl-6-pentyl-tétrahydro-α-pyrone, caractérisé en ce qu'on soumet la 3-méthyl-2-pentyl-cyclopentane-l-one à une oxydation de Bayer-Villiger.

8. Composition odorante et/ou aromatisante, caractérisée en ce qu'elle contient de la cis- ou de la trans-5-méthyl-6-pentyl-tétrahydro-α-pyrone, ou un mélange des deux isomères.

9. Composition odorante et/ou aromatisante
selon la revendication 8, caractérisée en ce qu'elle contient de la cis- ou de la trans-5-méthyl-6-pentyl-tétrahydro-α-pyrone, ou un mélange des deux isomères, à l'exception des compositions odorantes ou aromatisantes contenant ce dérivé naturel de la tétrahydro-α-pyrone.

10. Utilisation, en tant que matière odorante et/ou aromatisante, de la cis-5-méthyl-6-pentyl-tétrahydro-α-pyrone, de la trans-5-méthyl-6-pentyl-tétrahydro-α-pyrone ou d'un mélange des isomères cis/trans de la 5-méthyl-6-pentyl-tétrahydro-α-pyrone.

**Revendications pour l'Etat contractant suivant : ES**

1. Composition odorante et/ou aromatisante,
caractérisée en ce qu'elle contient de la cis- ou de la trans-5-méthyl-6-pentyl-tétrahydro-α-pyrone, ou un mélange des deux isomères.

2. Composition odorante et/ou aromatisante selon la revendication 1, caractérisée en ce qu'elle contient de la cis- ou de la trans-5-méthyl-6-pentyl-tétrahydro-α-pyrone, ou un mélange des deux isomères, à l'exception des compositions odorantes ou aromatisantes contenant ce dérivé naturel de la tétrahydro-α-pyrone.

3. Procédé pour préparer la 5-méthyl-6-pentyl-tétrahydro-α-pyrone, caractérisé en ce qu'on soumet la 3-méthyl-2-pentyl-cyclopentane-l-one à une oxydation de Bayer-Villiger.

4. Utilisation, en tant que matière odorante et/ou aromatisante, de la cis-5-méthyl-6-pentyl-tètrahydro-α-pyrone, de la trans-5-méthyl-6-pentyl-tétrahydro-α-pyrone ou d'un mélange des isomères cis/trans de la 5-méthyl-6-pentyl-tétrahydro-α-pyrone.